# EUROPEAN PATENT SPECIFICATION

(11) **EP 2 608 771 B1**
(45) Date of publication and mention of the grant of the patent: **12.12.2018**
(21) Application number: 11764342.9
(22) Date of filing: 18.08.2011
(51) Int. Cl.: A61K 9/08, A61K 38/26, A61K 38/28, A61K 9/00

(54) **COMPOSITIONS FOR GASTRIC DELIVERY OF ACTIVE AGENTS**
ZUSAMMENSETZUNGEN FÜR GASTRISCHE WIRKSTOFFFREISETZUNG
COMPOSITIONS POUR ADMINISTRATION GASTRIQUE D'AGENTS ACTIFS

(30) Priority: 23.08.2010 US 376060 P
(43) Date of publication of application: 03.07.2013
(73) Proprietor: Yissum Research Development Company of the Hebrew University of Jerusalem Ltd., 91390 Jerusalem (IL)
(72) Inventor: BEN-SASSON, Shmuel, 96343 Jerusalem (IL)
(74) Representative: Pearl Cohen Zedek Latzer Baratz UK LLP
(86) International application number: PCT/IL2011/000672
(87) International publication number: WO 2012/025921

(56) References cited:
- WO-A1-00/33866
- WO-A1-03/068186
- WO-A1-2008/145730
- WO-A2-2006/035416
- US-A1- 2005 171 193
- US-A1- 2007 249 546
- US-B1- 6 403 123
- Alison Brayfield (Ed.): "Pioglitazone Hydrochloride", Martindale: The Complete Drug Reference , 15 August 2014 (2014-08-15), pages 1-10, Retrieved from the Internet: URL:https://www.medicinescomplete.com/mc/m artindale/current/10380-l.htm?q=pioglitazo ne&t=search&ss=text&p=1#_hit [retrieved on 2015-07-06]

## Description

### FIELD OF THE INVENTION

This invention relates to pharmaceutical compositions enabling gastric absorption of active agents comprised therein.

### BACKGROUND OF THE INVENTION

Oral administration of biological macromolecules such as proteins, peptides, nucleic acids and glycosaminoglycans (GAGs) is considered the "*Holy Grail*" of biotechnology. The difficulty in the translocation of these macromolecules is twofold: (i) their large size prevents them from crossing the cell membrane and (ii) they can be readily degraded by a whole battery of digesting enzymes that exist in the gastrointestinal (GI) lumen. Although large molecules cannot cross the membrane of GI epithelial cells, some agents, like salts of medium-chain fatty acids, are able to disrupt the tight-junction structure, thereby possibly enabling macromolecules to cross the GI mucosal barrier, via the paracellular route (see Sugibayashi, K. et al. Europ. J. Pharm. Sci. 36: 246-253 (2009)).

It is noted that no viable technology known in the art was shown to achieve macromolecules delivery via the GI tract in a reasonable yield and reproducibility. Moreover, while there are some methods that claim intestinal absorption utilizing salts of medium-chain fatty acids (US 2007/0219131 and WO 2010/032140), they all apply to the large and small intestine and failed when tested for gastric absorption (Motlekar, NA et al. J. Drug Target. 13: 573-583 (2005)).

WO 00/33866 (Provalis UK Ltd.) discloses pharmaceutical compositions comprising insulin and optionally aprotinin, in a substantially non-aqueous hydrophilic medium comprising an alcohol and a cosolvent, in association with a medium chain partial glyceride, optionally in admixture with a long-chain PEG species.

### SUMMARY OF THE INVENTION

The present invention provides a composition comprising: at least one therapeutically active agent; at least one surface active agent being at least one of decanoic acid and lauric acid or any salt thereof; at least one ester of poly-carboxylic acid being triethyl-citrate; and a non-aqueous medium comprising propylene-glycol, wherein said at least one therapeutically active agent is a macromolecule having a MW of between 0.5kDa to 200kDa selected from a protein, peptide, lipid, nucleic acid, glycosaminoglycan and any combination thereof, or a hormone, growth factor, incretin, neurotrophic factor, anticoagulant, toxin, antibiotic, anti-fungal agent, antipathogenic agent, antigen, antibody, monoclonal antibody, antibody fragment, soluble receptor, immunomodulator, vitamin, antineoplastic agent, a cardiovascular agent, an antihypertensive agent, an agent used in the treatment of neurological disease, an agent capable of treating liver or kidney diseases, enzyme, gonadotropin, cytokine or any combination thereof.

In some embodiments, a composition of the invention is for use in the gastric absorption of said at least one therapeutically active agent.

In an embodiment, said at least one therapeutically active agent is selected from the group consisting of insulin, C-peptide, erythropoietin (EPO), glucagon-like peptide 1 (GLP-I), melanocyte stimulating hormone (ALPHA-MSH), parathyroid hormone (PTH), parathyroid hormone amino acids 1-34 (PTH(1-34)), growth hormone, peptide YY amino acids 3-36 (PYY(3-36)), calcitonin, interleukin-2 (IL-2), alpha1-antitrypsin, granulocyte/monocyte colony stimulating factor (GM-CSF), granulocyte colony stimulating factor (G-CSF), T20, monoclonal antibodies such as anti-TNF antibodies, interferon alpha, interferon beta, interferon gamma, luteinizing hormone (LH), follicle-stimulating hormone (FSH), enkephalin, dalargin, kyotorphin, basic fibroblast growth factor (bFGF), hirudin, hirulog, luteinizing hormone releasing hormone (LHRH), brain-derived natriuretic peptide (BNP), glatiramer acetate, coagulation factor VIII; coagulation factor IX, neurotrophic factors, human chorionic gonadotropin (hCG), tissue plasminogen activator (TPA), oxytocin, and combinations thereof.

In an embodiment, said at least one therapeutically active agent comprises at least 0.001 to 20% wt of the total weight of said composition.

In an embodiment, the composition further comprises at least one ester of an aromatic carboxylic acid.

In an embodiment, said at least one ester of poly-carboxylic acid comprises at least 5 to 90 %wt of total weight of said composition.

In an embodiment, the composition is for use in the oral administration of said at least one therapeutically active agent.

In an embodiment, the composition further comprising ethyl-benzoate.

### Therapeutically Active Agent

As used herein the term "*therapeutically active agent*" is meant to encompass any agent (either extracted from a natural source, a semi-synthetic compound or a synthetic compound) capable of facilitating any type of therapeutic response in a subject administered with said agent and in any quality or quantity.

Therapeutically active agents that can be used in a composition include any molecule or compound serving as, for example, a biological, therapeutic, pharmaceutical, or diagnostic agent including an imaging agent. The therapeutically active agents as defined herein include drugs and other agents including, but not limited to, those listed in the United States Pharmacopeia and in other known pharmacopeias.

In some embodiments said at least one therapeutically active agent has poor to negligible gastrointestinal absorption.

An agent having "*poor to negligible gastrointestinal absorption*" it should be understood to encompass an agent for which its gastrointestinal absorption through any part of the gastrointestinal tract is low, i.e. its oral bioavailability is less than about 5% or even less than about 1%, as compared to the agent's blood level when administered by injection. It is noted that due to this poor to negligible gastrointestinal absorption of said at least one therapeutic agent of the invention, such an agent does not confer any therapeutic effect when administered enterally, i.e. by oral, rectal, or sublingual administration.

Agents that have poor to negligible gastrointestinal absorption are unable to efficiently cross biological barriers, such as cell membrane or tight junctions. For example, such agents do not penetrate a biological membrane in an amount sufficient to achieve clinical efficacy.

In some embodiments said poor to negligible gastrointestinal absorbed agent includes but is not limited to: nucleic acids, glycosaminoglycans, proteins, peptides, polypeptides, polynucleotides, polysaccharides or pharmaceutically active agents, such as, for example, hormones, growth factors, incretins, neurotrophic factors, anticoagulants, bioactive molecules, toxins, antibiotics, anti-fungal agents, antipathogenic agents, antigens, antibodies, monoclonal antibodies, antibody fragments, soluble receptors, immunomodulators, vitamins, antineoplastic agents, enzymes, gonadotropins, cytokines, anti-hypertensive agents or other therapeutic agents.

In this invention, said at least one therapeutically active agent is a macromolecule. When referring to a "*macromolecule*" it should be understood to encompass any molecule (either extracted from a natural source, a semi-synthetic compound or a synthetic compound) having a molecular weight of between about 0.5kDa to about 200kDa.

In further embodiments said macromolecule has a molecular weight of about 0.5kDa, 1kDa, 1.5kDa, 2 kDa, 2.5 kDa, 3 kDa, 3.5 kDa, 4 kDa, 4.5 kDa, 5 kDa, 5.5 kDa, 6 kDa, 6.5 kDa, 7 kDa, 7.5 kDa, 8 kDa, 8.5 kDa, 9 kDa, 9.5 kDa, 10 kDa, 15 kDa, 20 kDa, 25 kDa, 30 kDa, 35 kDa, 40 kDa, 45 kDa, 50 kDa, 55 kDa, 60 kDa, 65 kDa, 70 kDa, 75 kDa, 80 kDa, 85 kDa, 90 kDa, 95 kDa, 100 kDa 110 kDa, 120 kDa, 130 kDa, 140 kDa, 150 kDa, 160 kDa, 170 kDa, 180kDa, 190kDa, 200kDa. In yet other embodiments said macromolecule has a molecular weight below 2kDa.

In other embodiments said macromolecule is selected from protein, peptide, lipid, nucleic acid, glycosaminoglycan or any combination thereof.

In some embodiments, glycosaminoglycans include, but are not limited to, heparin, heparin derivatives, heparin sulfate, chondroitin sulfate, dermatan sulfate, and hyaluronic acid. In further embodiments, heparin derivatives include, but are not limited to, low molecular weight heparins such as enoxaparin, dalteparin, tinzaparin, and fondaparinux. In other embodiments, nucleic acids include, but are not limited to, specific DNA sequences (e.g., coding genes), specific RNA sequences (e.g., RNA aptamers, antisense RNA, siRNA, or a specific inhibitory RNA (RNAi)), poly CpG, or poly I:C synthetic polymers of nucleic acids. In further embodiments proteins include, but are not limited to, insulin, C-peptide, erythropoietin (EPO), glucagon-like peptide 1 (GLP-I) and analogs thereof such as Exenatide, melanocyte stimulating hormone (ALPHA-MSH), parathyroid hormone (PTH), parathyroid hormone amino acids 1-34 (PTH(1-34)), growth hormone, peptide YY amino acids 3-36 (PYY(3-36)), calcitonin, interleukin-2 (IL-2), alpha1-antitrypsin, granulocyte/monocyte colony stimulating factor (GM-CSF), granulocyte colony stimulating factor (G-CSF), T20, monoclonal antibodies such as anti-TNF antibodies, interferon alpha, interferon beta, interferon gamma, luteinizing hormone (LH), follicle-stimulating hormone (FSH), enkephalin, dalargin, kyotorphin, basic fibroblast growth factor (bFGF), hirudin, hirulog, luteinizing hormone releasing hormone (LHRH) and analogs thereof that serve either as agonists or antagonists, brain-derived natriuretic peptide (BNP), glatiramer acetate, coagulation factor VIII; coagulation factor IX; and neurotrophic factors, human chorionic gonadotropin (hCG), tissue plasminogen activator (TPA), oxytocin, and analogs and combinations thereof. Additional pharmaceutically active agents may be selected from the group consisting of vitamin B12, a bisphosphonate (e.g., disodium pamidronate, alendronate, etidronate, tiludronate, risedronate, zoledronic acid, sodium clodronate, and ibandronic acid), taxol, Caspofungin, or an aminoglycoside antibiotic.

In other embodiments of the invention said at least one therapeutically active agent is selected from a hormone, growth factor, incretin, neurotrophic factor, anticoagulant, toxin, antibiotic, anti-fungal agent, antipathogenic agent, antigen, antibody, monoclonal antibody, antibody fragment, soluble receptor, immunomodulator, vitamin, antineoplastic agent, a cardiovascular agent, antihypertensive agent, an agent used in the treatment of neurological disease, an agent capable of treating liver or kidney diseases, enzyme, gonadotropin, cytokine, or any combination thereof.

In some embodiments of the present invention, said at least one therapeutically active agent is selected from the group consisting of insulin, C-peptide, erythropoietin (EPO), glucagon-like peptide 1 (GLP-I), melanocyte stimulating hormone (ALPHA-MSH), parathyroid hormone (PTH), parathyroid hormone amino acids 1-34 (PTH(1-34)), growth hormone, peptide YY amino acids 3-36 (PYY(3-36)), calcitonin, interleukin-2 (IL-2), alpha1-antitrypsin, granulocyte/monocyte colony stimulating factor (GM-CSF), granulocyte colony stimulating factor (G-CSF), T20, monoclonal antibodies such as anti-TNF antibodies, interferon alpha, interferon beta, interferon gamma, luteinizing hormone (LH), follicle- stimulating hormone (FSH), enkephalin, dalargin, kyotorphin, basic fibroblast growth factor (bFGF), hirudin, hirulog, luteinizing hormone releasing hormone (LHRH), brain-derived natriuretic peptide (BNP), glatiramer acetate, coagulation factor VIII; coagulation factor IX, neurotrophic factors, human chorionic gonadotropin (hCG), tissue plasminogen activator (TPA), oxytocin, including any analogs thereof and any combinations thereof.

In other embodiments of the invention said at least one active agent is a polyanionic compound.

In some embodiments of the invention said at least one active agent is heparin. In some embodiments of the invention said at least one active agent is insulin.

In further embodiments of a composition of the invention, said at least one therapeutically active agent comprises at least about 0.001 to about 20%wt of total weight of said composition. In other embodiments of a composition of the invention, said at least one therapeutically active agent comprises at least about 0.001, 0.0015, 0.01, 0.015, 0.1, 0.15, 0.2, 0.5, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10, 11, 12, 13, 14, 15, 16, 17, 18, 19, 20%wt of total weight of said composition

### Surface Active Agent

The term "*surface active agent*" or "*surfactant*", used interchangeably herein, should be understood to encompass any agent that is capable of lowering the surface tension of a liquid, allowing for the formation of a homogeneous mixture of at least one type of liquid with at least one other type of liquid, or between at least one liquid and at least one solid. In this invention, the at least one surface active agent is at least one of decanoic acid and lauric acid or any salt thereof

In other embodiments of a composition of the invention, said at least one surface active agent comprises at least about 2 to about 20 %wt of total weight of said composition. In other embodiments of a composition of the invention, said at least one surface active agent comprises at least about 2.0, 2.5, 3.0, 3.5, 4.0, 4.5, 5.0, 5.5, 6.0, 6.5, 7.0, 7.5, 8.0, 8.5, 9.0, 9.5, 10, 11, 12, 13, 14, 15, 16, 17, 18, 19, 20%wt of total weight of said composition.

### Non-Aqueous Medium

The above noted components of a composition of the invention are mixed into a non-aqueous medium, i.e. in a medium that does not comprise water. In this invention, the non-aqueous medium comprises propylene-glycol.

In embodiments of a composition of the invention, said propylene-glycol comprises at least about 10 to about 90 %wt of total weight said composition. In further embodiments of a composition of the invention, said propylene-glycol comprises at least about 10, 15, 20, 25, 30, 35, 40, 45, 50, 55, 60, 65, 70, 75, 80, 85 to about 90 %wt of total weight said composition.

### Esters of poly carboxylic acids

As used herein the term "*esters of poly carboxylic acid*" should be understood to encompass a compound having multiple (at least 2, in some embodiments between 2, 3, to 4) independent ester groups, i.e. -C(=O)OR groups, originating from the corresponding poly-carboxylic acids (having multiple -C(=O)OH groups substituted on an aliphatic, aromatic or hereroaromatic back-bone which may be optionally further substituted). In this invention, the at least one ester of poly-carboxylic acid comprises triethyl-citrate.

In further embodiments of a composition of the invention, said at least one ester of poly-carboxylic acid comprises at least about 5 to about 90 %wt of total weight of said composition. In other embodiments of a composition of the invention, said at least one ester of poly-carboxylic acid comprises at least about 5, 10, 15, 20, 25, 30, 35, 40, 45, 50, 55, 60, 65, 70, 75, 80, 85 to about 90 %wt of total weight of said composition.

### An ester of an aromatic carboxylic acid

As used herein the term "*esters of aromatic carboxylic acid*" is meant to encompass a compound having an ester group, i.e. -C(=O)OR group, originating from an aromatic carboxylic acid reacted with aliphatic C₁-C₁₀ straight or branched mono-alcohol (providing the group R on said ester group, wherein R is a C₁-C₁₀ straight or branched alkyl of the corresponding mono alcohol), substituted on an aliphatic straight, branched or cyclic C₁-C₁₀ alkyl, alkenyl or alkynyl, aromatic or heteroaromatic back-bone, which may be further substituted by at least one substituent selected from halogen, hydroxy, cyano, amino or nitro. Non-limiting examples of esters of benzoic acid (=an aromatic carboxylic acid) that may be used as flavor ingredients: Methyl-, Ethyl-, Propyl-, Hexyl-, Isopropyl-, Isobutyl-, Isoamyl-, cis-3-hexenyl-, Linalyl- and Geranyl-Benzoate (Adams TB at al. Food and Chemical Toxicology 43: 1207-40 (2005)).

In some embodiments, a composition of the invention further comprises at least one ester of an aromatic carboxylic acid.

In further embodiments, said ester of an aromatic carboxylic acid is formed by reacting an aromatic carboxylic acid with at least one mono-alcohol.

In some embodiments said at least one alcohol is selected from C₁-C₈ aliphatic alcohol, C₃-C₈ alicyclic alcohol, C₆-C₁₀ aromatic alcohol or any combinations thereof.

In other embodiments, said ester of an aromatic carboxylic acid is an ester of benzoic acid.

In further embodiments of a composition of the invention, said at least one ester of an aromatic carboxylic acid comprises at least about 5 to about 90 %wt of total weight of said composition. In other embodiments of a composition of the invention, said at least one ester of an aromatic carboxylic acid comprises at least about 5, 10, 15, 20, 25, 30, 35, 40, 45, 50, 55, 60, 65, 70, 75, 80, 85 to about 90 %wt of total weight of said composition.

### Compositions

In some embodiments a composition of the invention is a pharmaceutical composition. In some embodiments said pharmaceutical composition is an enteral administered composition, i.e. intended for oral, sublingual, rectal administration. In other embodiments, a pharmaceutical composition of the invention is suitable for oral, buccal, anal, rectal, bronchial, pulmonary, nasal, sublingual, intraorbital, transmucosal, administration. In other embodiments, said pharmaceutical composition is an orally administered pharmaceutical composition.

It is disclosed herein that the composition of the invention is for use in therapy.

In another aspect the invention provides a composition of the invention for use in the oral administration of said at least one therapeutically active agent.

In some embodiments of the invention, a composition of the invention comprises: at least one therapeutically active agent; at least one of decanoic acid (comprising about ∼2.5%wt of the total weight of the composition) and lauric acid (comprising about ∼2.5%wt of the total weight of the composition) or any salt thereof; propylene-glycol (comprising about ∼45%wt of the total weight of the composition); triethyl-citrate (comprising about 50%wt of the total weight of the composition). In other embodiments a composition of the invention further comprises ethyl-benzoate (comprising about -5% to 50%wt of the total weight of the composition, replacing an equivalent weight amount of triethyl-citrate).

Salts encompassed within the term "*pharmaceutically acceptable salts*" refer to non-toxic salts of the compounds defined in this invention, which are generally prepared by reacting the free base with a suitable organic or inorganic acid or solvent to produce "*pharmaceutically-acceptable acid addition salts*" of the compounds described herein. These compounds retain the biological effectiveness and properties of the free bases Representative examples of such salts include the water-soluble and water-insoluble salts, such as the acetate, amsonate (4,4-diaminostilbene-2,2'-disulfonate), benzenesulfonate, benzoate, bicarbonate, bisulfate, bitartrate, borate, bromide, butyrate, calcium edetate, camsylate, carbonate, chlonde, citrate, clavulapiate, dihydrochlopide, edetate, edisylate, estolate, esylate, fumarate, gluceptate, gluconate, glutamate, glycouylarsanilate, hexafluorophosphate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methyhiitrate, methylsulfate, mucate, napsylate, nitrate, N-methylglucamine ammonium salt, 3-hydroxy-2-naphthoate, oleate, oxalate, palmitate, pamoate (1,1- methylene-bis-2-hydroxy-3-naphthoate, embonate), pantothenate, phosphate/diphosphate, picrate, polygalacturonate, propionate, p-toluenesulfonate, salicylate, stearate, subacetate, succinate, sulfate, sulfosaliculate, suramate, tannate, tartrate, teoclate, tosylate, triethiodide, and valerate salts.

### Uses of the invention

In one aspect of the invention, there is provided a composition comprising: at least one therapeutically active agent; at least one surface active agent; at least one ester of poly-carboxylic acid; and a non-aqueous medium; as claimed, for use in the gastric absorption of said at least one therapeutically active agent.

In a further aspect, the invention encompasses a composition comprising: at least one therapeutically active agent; at least one surface active agent; at least one ester of poly-carboxylic acid; and a non-aqueous medium; as claimed, for use in the oral administration of said at least one therapeutically active agent.

In some embodiments of a use of the invention, said composition further comprises at least one ester of an aromatic carboxylic acid.

In yet another one of its aspects the invention provides a composition of the invention for use in the treatment of a disease or disorder in a subject in need thereof.

The invention further relates to a composition of the invention for use in treating or preventing diseases, and/or disorders and/or pathological conditions in a subject in which such treatment or prevention is desired.

For example, diseases, disorders or conditions to be treated include, but are not limited to, endocrine disorders, including diabetes, infertility, hormone deficiencies and osteoporosis; ophthalmological disorders; neurodegenerative disorders, including Alzheimer's disease and other forms of dementia, Parkinson's disease, multiple sclerosis, and Huntington's disease; cardiovascular disorders, including atherosclerosis, hyper- and hypocoagulable states, coronary disease, and cerebrovascular events; hypertension, metabolic disorders, including obesity and vitamin deficiencies; renal disorders, including renal failure; haematological disorders, including anemia of different entities; immunologic and rheumatologic disorders, including autoimmune diseases, and immune deficiencies; inflammatory diseases, infectious diseases, including viral, bacterial, fungal and parasitic infections; neoplastic diseases; and multifactorial disorders, including impotence, chronic pain, depression, different fibrosis states, and short stature.

### Routes of administration and dosage formulations

Administration of a composition of the invention can be via any of the accepted modes of administration for therapeutic agents to be delivered through the gastrointestinal tract.

These routes include oral, buccal, anal, rectal, sublingual administration modes. The oral dosage form of a composition of the invention may be in the form (formulated) of, creams, ointments, pills, tablets, soft-gel capsules, time-release capsules, liquids, suspensions, preferably in unit dosages. A composition of the invention will include an effective amount of therapeutically active agent or a pharmaceutically acceptable salt thereof, and in addition, may also include any conventional pharmaceutical excipients and other medicinal or pharmaceutical drugs or agents, carriers, adjuvants, diluents, protease inhibitors, etc., as are customarily used in the pharmaceutical sciences.

If desired, a pharmaceutical composition to be administered may also contain pharmaceutically acceptable auxiliary agents, such as for example wetting or emulsifying agents, pH buffering agents, and other substances such as for example, sodium acetate, triethanolamine oleate, etc.

Those skilled in the art will recognize that the compositions of the present invention can also be used for mucosal vaccination, i.e., an oral, transmucosal, rectal, or vaginal, vaccine comprising an antigen, towards which an immune response is desired, which serves as the active agent in a composition of the invention. Such a vaccine can include a composition including a desired antigenic sequence, including, but not limited to, the protective antigen (PA) component of Anthrax, or the Hepatitis B surface antigen (HBs) of Hepatitis B. This composition can then be orally or nasally administered to a subject in need of vaccination. The composition for mucosal vaccination can be administered to humans and also to other animals. These are referred to in general as "subjects" or "patients". Such animals include farm animals such as cattle, sheep, goats, horses, chickens, and also cats, dogs, and any other animal in veterinary care.

Generally it is noted that the term "*...at least one...* " as applied to any component of a composition of the invention should be read to encompass one, two, three, four, five, six, seven, eight, nine or ten different occurrences of said component in a composition of the invention.

It must be noted that, as used in this specification and the appended claims, the singular forms "*a*"*,* "*an*" and "*the*" include plural referents unless the content clearly dictates otherwise.

Throughout this specification and the claims which follow, unless the context requires otherwise, the word "*comprise*", and variations such as "*comprises*" and "*comprising*", will be understood to imply the inclusion of a stated integer or step or group of integers or steps but not the exclusion of any integer or step or group of integers and steps.

### BRIEF DESCRIPTION OF THE DRAWINGS

In order to understand the invention and to see how it may be carried out in practice, embodiments will now be described, by way of non-limiting example only, with reference to the accompanying drawings, in which:

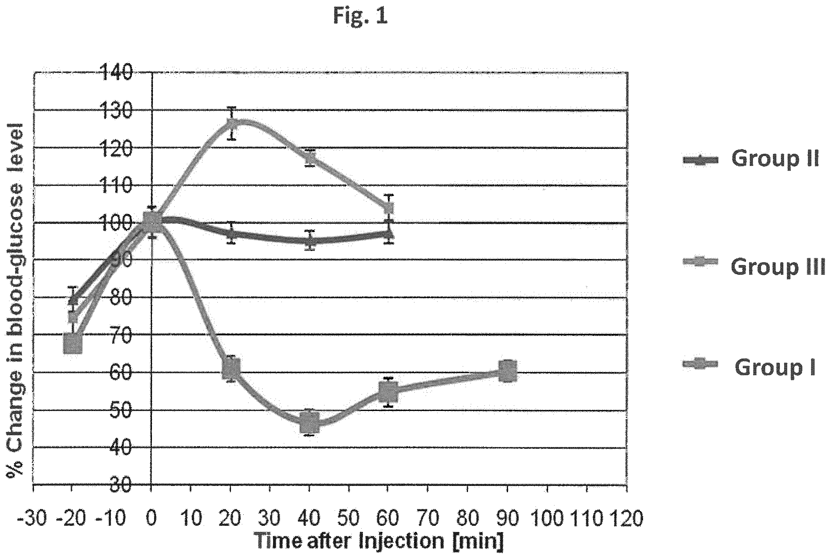

**Fig. 1** shows the change in blood-glucose level, as indicated in Example 1, relative to time 0', of Groups I, II and III were rats in each group were treated with composition I, II or III.

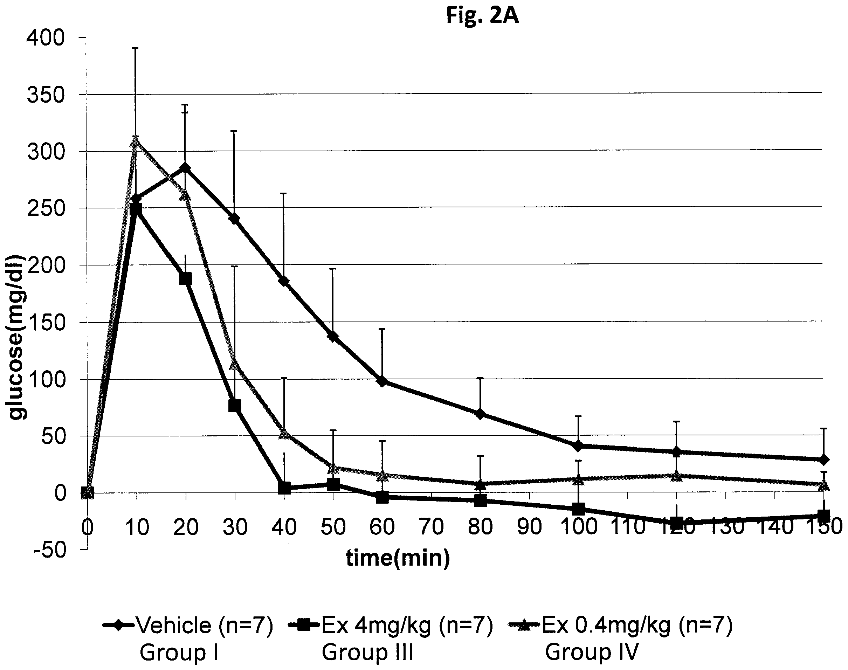

**Fig. 2A** shows the change in blood-glucose level, as indicated in Example 4, relative to time 0', following IPGTT, in Group I (orally administered vehicle composition), Group III (orally administered 4mg/kg Exenatide composition), and Group IV (orally administered 0.4mg/kg Exenatide composition).

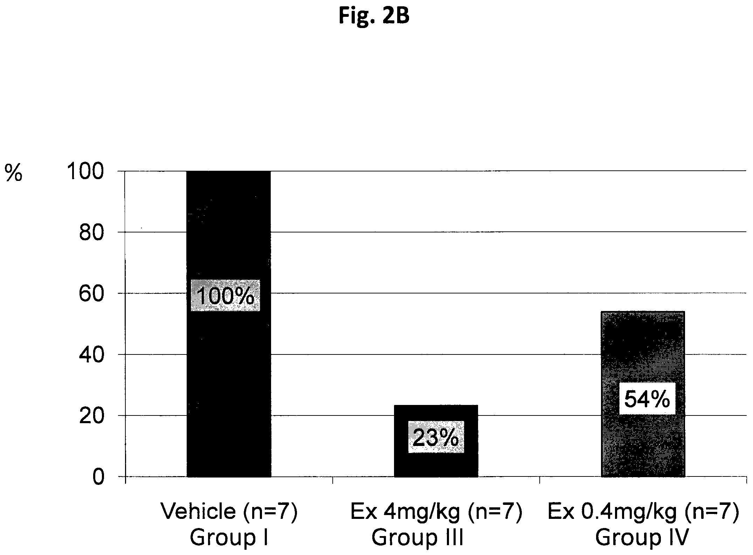

**Fig. 2B** shows the relative AUC of blood-glucose level results of Groups I, III and IV in Fig. 2A above.

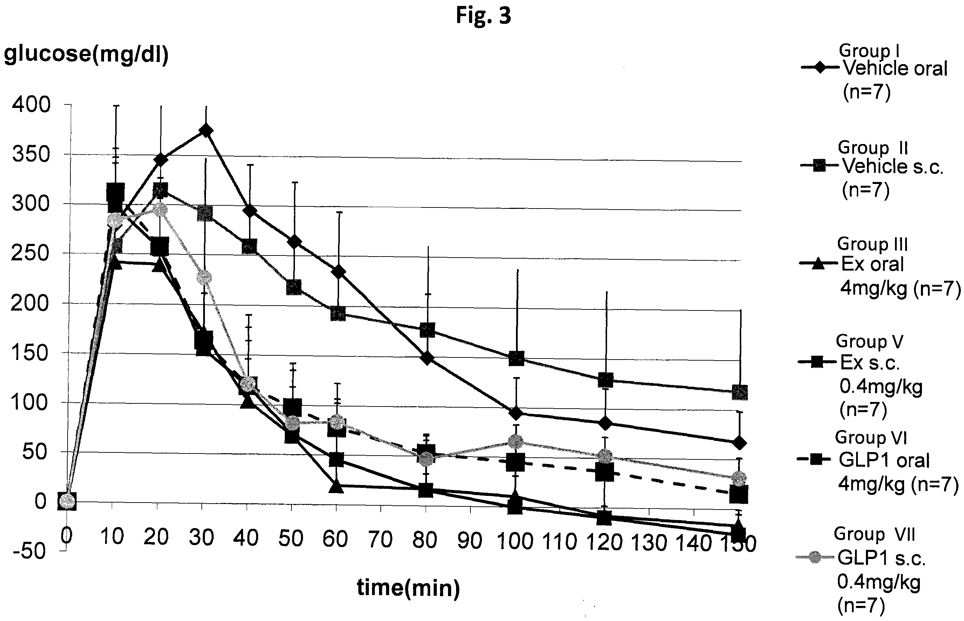

**Fig. 3** shows the change in blood-glucose level, as indicated in Example 4, relative to time 0', in Group I (orally administered vehicle composition), Group II (s.c. administered vehicle composition), Group III (orally administered 4mg/kg Exenatide composition), Group V (s.c. administered 0.4mg/kg Exenatide composition), Group VI (orally administered 4mg/kg GLP-1 composition), Group VII (s.c. administered 0.4mg/kg GLP-1 composition).

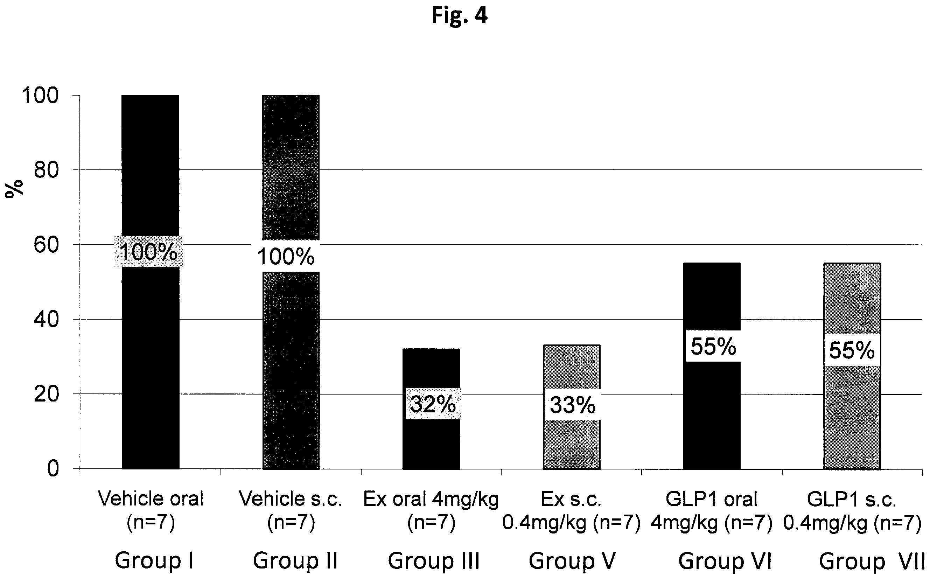

**Fig. 4** shows the AUC glucose level as indicated in Example 4, relative to time 0', in Group I (orally administered vehicle composition), Group II (s.c. administered vehicle composition), Group III (orally administered 4mg/kg Exenatide), Group V (s.c. administered 0.4mg/kg Exenatide composition), Group VI (orally administered 4mg/kg GLP-1 composition), Group VII (s.c. administered 0.4mg/kg GLP-1 composition).

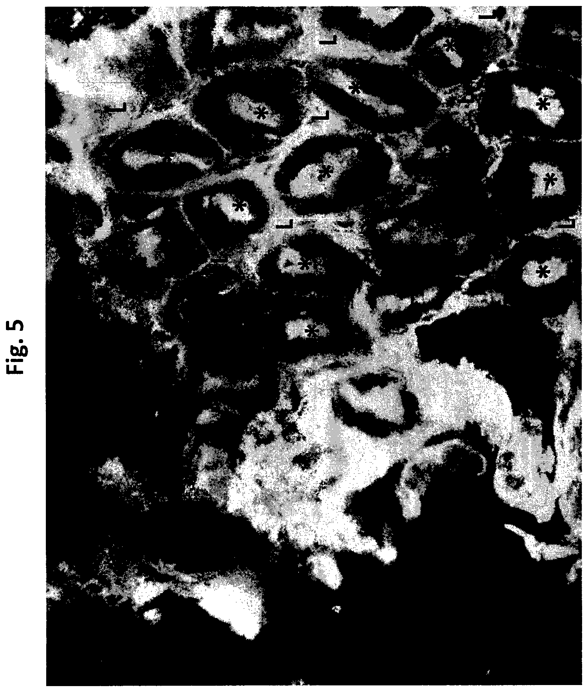

**Fig. 5** shows the cross-section through the gastric pits following gavage administration of a composition of the invention comprising FITC-Dextran as therapeutically active agent. The figure depicts translocation of the FITC-Dextran from pits' lumen (marked with *) to the lamina propria (marked with **L**) where blood vessels are located.

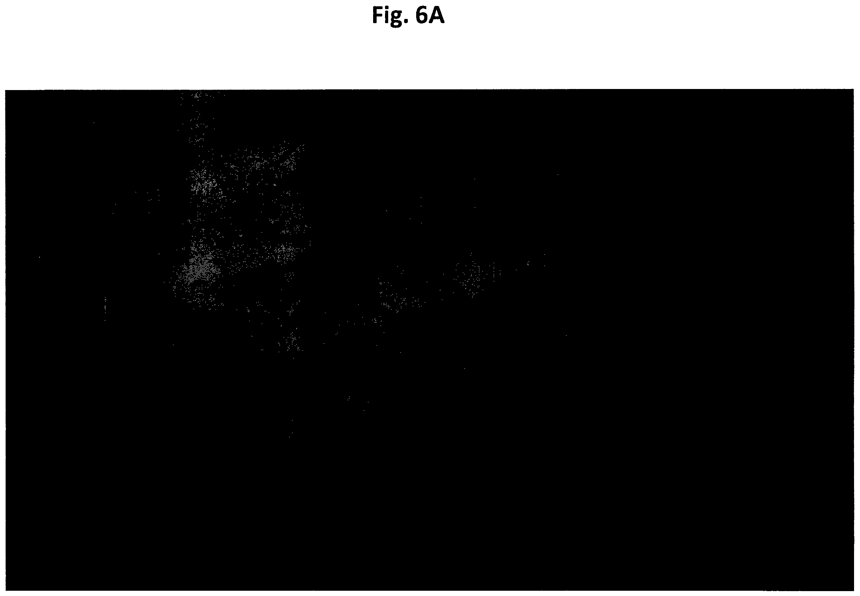

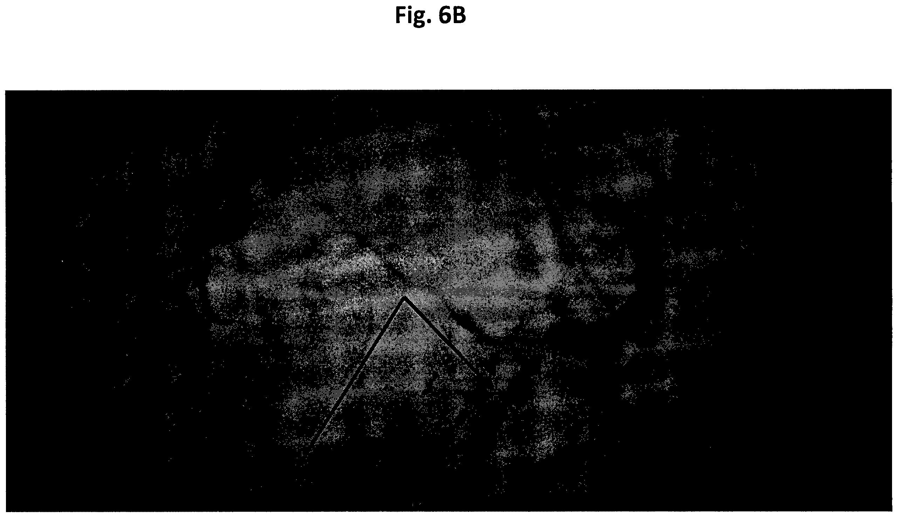

**Figs. 6A-6B** show the external surface of the stomach via fluorescence microscopy of non-formulated FITC-Albumin (PBS-dissolved, Fig. 6A) and following gavage administration of composition of the invention comprising FITC-Albumin (-64 KDa) as therapeutically active agent (Fig. 6B), depicting the influx of the fluorescent protein from the gastric lumen into gastric-draining blood vessels.

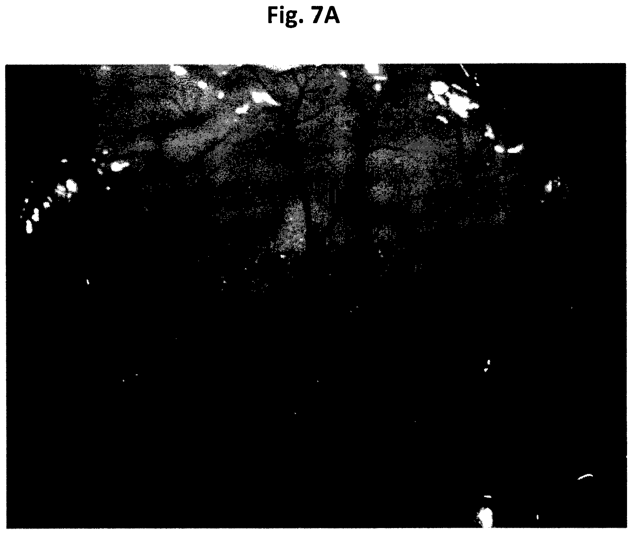

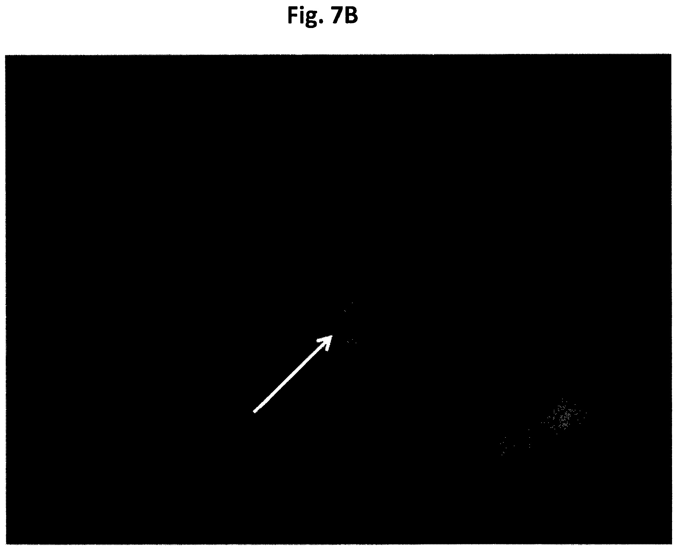

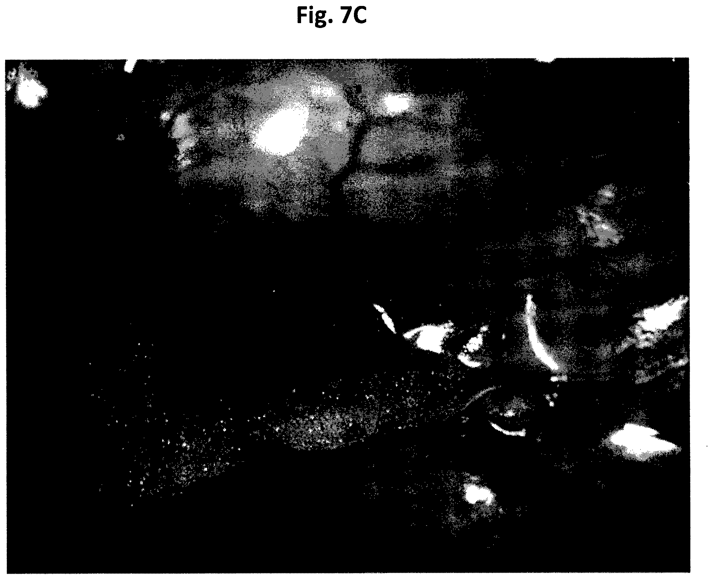

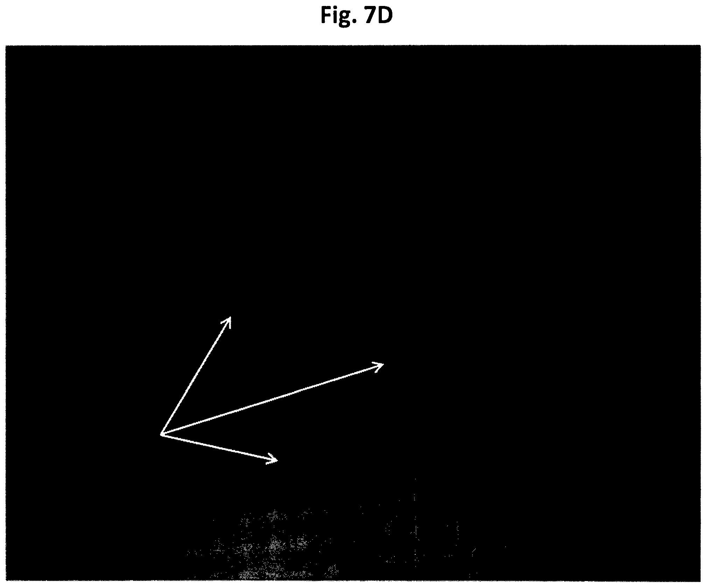

**Figs. 7A-7D** shows the external side of small-intestine loops following administration of composition of the invention comprising FITC-Albumin (-64 KDa) as therapeutically active agent, by gavage. Figs. 7A (showing the lymphatic nodule) and 7C (showing the lymphatic vessels) are light microscopy figures, and Figs. 7B and 7D are corresponding fluorescent microscopy figures.

### DETAILED DESCRIPTION OF EMBODIMENTS

### Example 1: Compositions comprising h-Insulin as therapeutically active agent

### Compounds:

Recombinant human insulin [Sigma, 12643]
Propylene Glycol (PG) [Sigma, P-4347]
Triethyl citrate (TEC) [Aldrich, 109290]
Glyceryl tributyrate (GTB) [Sigma, T8626]
Sodium Laurate (=dodecanoate) [Sigma, L9755]
Sodium Caprate (=decanoate) [Sigma, C4151]
SPAN40 [Spectrum, SPA43]
GMO [Abitec Corp. Capmul GMO-50]
Ethyl-Isovalerate [Sigma, 112283]
Lecithin [Spectrum, L1083]
1-Octanol [Sigma, 112615]
Geraniol [Sigma, 163333]

### Contents and Preparation of Mixture "1":

2 g Sodium decanoate
2 g Sodium laurate
36 ml PG

All the above components (Sodium decanoate, Sodium laurate and PG) were mixed (vortexed) and heated (60°C) to dissolution. The resultant mixture "*1*" was than stored at 4°C.

### Contents and Preparation of Mixture "2":

12 ml 1-Octanol
12 ml Geraniol
12 ml Ethyl Isovalerate
2 ml GMO

All the above components (1-octanol, geraniol, Ethyl isovalerate, GMO) were mixed to homogeneity. 2 g Span40 were then added, mixed and heated (60°C) to dissolution. Upon dissolution 8 g Lecithin were added, vortexed and heated (60°C) to dissolution. The resultant mixture "2" was stored at 4°C.

### Preparation of Composition "I" for gastric administration:

In 15ml test-tube, 20 mg h-Insulin was dissolved in 2.5 ml of mixture "*1*" by heating to 60°C for ∼2 hrs (intermittent mixing, as usual). 2.5 ml TEC, were then added and mixed in a rotary-shaker at 37°C, 60 rpm, for 5 hrs. The composition was stored at RT.

### Preparation of Composition "II" for gastric administration:

In 15ml test-tube, 20 mg h-Insulin was dissolved in 2.5 ml mixture "*1*" by heating to 60°C for ∼2 hrs (intermittent mixing, as usual). 2.5 ml GTB were then added and mixed in a rotary-shaker at 37°C, 60 rpm, for 5 hrs. The composition was stored at RT.

### Preparation of Composition "III" for gastric administration:

In 15ml test-tube, 20 mg h-Insulin were dissolved in 2.5 ml mixture "*1*" by heating to 60°C for ∼2 hrs (intermittent mixing, as usual). 2.5 ml of mixture "*2*" were then added, and mixed in a rotary-shaker at 37°C, 60 rpm, for 5 hrs. The composition was stored at RT.

**Animals:** 15 SD/Hsd male rats weighing 175-200g.

### Experimental Groups:

Group I: 5 rats treated with composition I (0.1ml) at time 0'.
Group II: 5 rats treated with composition II (0.1ml) at time 0'.
Group III: 5 rats treated with composition III (0.1ml) at time 0'.

**Administration:** Gavage 0.1 ml per rat.

**Monitoring of active-insulin translocation:** Measurement of blood glucose concentration (mg/dl) was performed using Contour glycometers [Bayer]. Measurements of blood glucose concentration was initiated 20min prior to administration and was monitored every 20min (at times -20min, 0, 20min, 40min, 60min, for Group III a further measurement at 90min was also conducted).

### Results:

Composition I represents an embodiment composition of the invention.
Composition II represents a control composition were a triglyceride (GTB) was used having similar molecular weight as TEC.
Composition III represents a control composition were TEC was replaced by a mixture of compounds that were reported to facilitate absorption through the large and small intestine (WO2006/097793 and WO2010/032140).

**Fig. 1** shows the change in blood-glucose level in each experimental group over a period of 110min (the compositions I, II and III were administered to each respective group at time 0').

In view of the change in blood-glucose level in each experimental group it is concluded that:
- Composition III was substantially inactive and the rise in blood glucose level reflected the induced stress following the intragastric administration.
- Composition II was weakly active, and was able to compensate for the rise in glucose due to the stress.
- Composition I showed the most robust, sustained drop in blood-glucose level due to the rapid translocation of insulin from the gastric lumen into the bloodstream.

### Example 2: Composition of the invention comprising Glucagon-like Peptide-1 (GLP1) as therapeutically active agent

### Compounds:

Propylene Glycol (**PG**) [Sigma, P-4347]
Triethyl citrate (**TEC**) [Aldrich, 109290]
Sodium Laurate (=dodecanoate) [Sigma, L9755]
Sodium Caprate (=decanoate) [Sigma, C4151]
Aerosil 200 Silicon dioxide (**Aerosil**) [Evonic Industries (past Degussa AG)]
Glucagon-like Peptide-1 (**GLP1**), solid-phase synthesis by Novetide, Israel

### Contents and Preparation of Mixture "I":

2 g Sodium decanoate
2 g Sodium laurate
36 ml PG

All the above components (Sodium decanoate, Sodium laurate and PG) were mixed (vortexed) and heated (60°C) to dissolution. The resultant mixture "*1*" was than store at 4°C.

### Preparation of GLP-1 Composition for gastric administration:

In 15ml test-tube, 5 mg GLP1 were dissolved in 2.5 ml of mixture "*I*" by heating to 60°C for ∼2 hrs (intermittent mixing, as usual). 2.5 ml TEC and 100 mg Aerosil were then added and mixed in a rotary-shaker at 37°C, 60 rpm, for 5 hrs. The composition was stored at RT.

### Example 3: Composition of the invention comprising Exenatide as therapeutically active agent

### Compounds:

Propylene Glycol (**PG**) [Sigma, P-4347]
Triethyl citrate (**TEC**) [Aldrich, 109290]
Sodium Laurate (=dodecanoate) [Sigma, L9755]
Sodium Caprate (=decanoate) [Sigma, C4151]
Aerosil 200 Silicon dioxide (**aerosil**) [Evonic Industries (past Degussa AG)]
Exenatide (**Exenatide**), solid-phase synthesis by Novetide, Israel

### Contents and Preparation of Mixture "I":

2 g Sodium decanoate
2 g Sodium laurate
36 ml PG

All the above components (Sodium decanoate, Sodium laurate and PG) were mixed (vortexed) and heated (60°C) to dissolution. The resultant mixture "*1*" was than stored at 4°C.

### Preparation of Exenatide Composition for gastric administration:

In 15ml test-tube, 5 mg Exenatide were dissolved in 2.5 ml of mixture "*1*" by heating to 60°C for ~2 hrs (intermittent mixing, as usual). 2.5 ml TEC and 100 mg Aerosil were then added and mixed in a rotary-shaker at 37°C, 60 rpm, for 5 hrs. The composition was stored at RT.

### Example 4: In vitro results of GLP-1 composition (prepared according to Example 2) and Exenatide composition (prepared according to Example 3)

**Animals:** C57BL/6 male mice, 8-9 wks old (∼25g body weight).

### Vehicle Composition - Contents and preparation

### Compounds:

Propylene Glycol (**PG**) [Sigma, P-4347]
Triethyl citrate (**TEC**) [Aldrich, 109290]
Sodium Laurate (=dodecanoate) [Sigma, L9755]
Sodium Caprate (=decanoate) [Sigma, C4151]
Aerosil 200 Silicon dioxide (**aerosil**) [Evonic Industries (past Degussa AG)]

### Contents and Preparation of Mixture "I":

2 g Sodium decanoate
2 g Sodium laurate
36 ml PG

All the above components (Sodium decanoate, Sodium laurate and PG) were mixed (vortexed) and heated (60°C) to dissolution. The resultant mixture "*1*" was then stored at 4°C.

### Preparation of Vehicle composition:

2.5 ml of mixture "*1*" were mixed with 2.5 ml TEC and 100 mg Aerosil. This mixture was than vortexed and stored at RT.

### Experimental Groups:

Group I: 7 mice orally administered with 0.1ml vehicle composition.
Group II: 7 mice s.c. administered with 0.1ml - PBS (=s.c. vehicle).
Group III: 7 mice orally administered with 0.1ml with 4mg/kg Exenatide composition (prepared as disclosed in Example 3).
Group IV: 7 mice orally administered with 0.1ml with 0.4mg/kg Exenatide composition (prepared as disclosed in Example 3).
Group V: 7 mice s.c. administered with 0.1ml with 0.4mg/kg Exenatide composition (prepared as disclosed in Example 3).
Group VI: 7 mice orally administered with 0.1ml with 4mg/kg GLP-1 composition (prepared as disclosed in Example 2).
Group VII: 7 mice orally administered with 0.1ml with 0.4mg/kg GLP-1 composition (prepared as disclosed in Example 2).

It is noted that Groups V, and VII administered s.c. with one tenth of the oral dose (Groups III and VI) were performed for comparison purposes in order to enable estimation of bioavailability relative to the current mode of s.c. administration to patients (see: http://pi.lilly.com/us/byetta-pi.pdf).

### Administration:

Oral administration: Gavage 1 mg/ml preparation of each composition, as specified above, 0.1 ml per mouse.

Subcutaneous administration: 1 mg/ml preparation of each composition, as specified above was dissolved in PBS, s.c. injection of 0.1 ml.

### Intra-peritoneal glucose tolerance-test (IPGTT) protocol:

To minimize the stress-effect, blood-glucose level was tested at time: -20', -10' and 0'. IPGTT was executed immediately after time 0' blood-glucose measurement by i.p. injection of 0.14 ml of 25% glucose aqueous solution. The gavage or s.c. composition was administered 5' after IPGTT injection. For further calculations, time 0' blood-glucose level is the average of the -10' and 0' samples.

### Monitor of therapeutically active agent (GLP or Exenatide) absorption via the enhanced clearance of glucose following IPGTT:

Measurement of blood glucose concentration (mg/dl) was performed using Contour glycometers [Bayer]. For bioavailability quantification, the results are expressed as the area under the curve (AUC) of excessive blood glucose level, above time 0' baseline. The results obtained following gavage administration of the peptide (therapeutically active agent: GLP1 in composition of Example 2 or Exenatide in composition of Example 3) were compared to the vehicle-only, administered by gavage (=100% reference). This change in AUC is then compared to the AUC obtained by s.c. injection of the peptide.

### Results:

Fig. 2A shows the change in blood-glucose level, relative to time 0', following IPGTT, in Groups I (orally administered vehicle composition), III (orally administered 4mg/kg Exenatide composition of Example 3), and IV (orally administered 0.4mg/kg Exenatide composition of Example 3). The compositions were administered at time 5' (IPGTT injection was administered at time 0'). Fig. 2A demonstrates the dose-response in the enhanced clearance of glucose from the blood-stream following an oral administration of Exenatide composition. Fig. 2B shows the relative AUC calculations of blood-glucose level of Groups I, III and IV above.

Fig. 3 shows the change in blood-glucose level, relative to time 0', following IPGTT, in Groups I (orally administered vehicle composition), II (s.c. administered vehicle composition), III (orally administered 4mg/kg Exenatide composition of Example 3), V (s.c. administered 0.4mg/kg Exenatide composition of Example 3), VI (orally administered 4mg/kg GLP-1 composition of Example 2), VII (s.c. administered 0.4mg/kg GLP-1 composition of Example 2). The compositions were administered at time 5' (IPGTT injection was done at time 0'). For more details see Examples 2 and 3 below.

Fig. 4 summarizes the results of the experiments depicted in Fig. 3. The calculated mean AUC is shown for each treatment group, presented as % compared to the corresponding control. As can be seen, oral administration of GLP-1 composition and oral administration of Exenatide composition have an efficacy that is almost identical to that of one tenth of the corresponding peptide when injected s.c. Thus, the oral composition yields about 10% bioavailability for both peptides.

Figs. 3 and 4 show that both Exenatide and GLP-1 when administered orally in compositions of the present invention (as prepared in Examples 2 and 3), enhance the clearance of blood-glucose level following IPGTT demonstrating bioavailability of around 10%.

### Example 5: Demonstration of GI crossing of compositions comprising FITC-Dextran and FITC-Albumin

### Compounds:

Propylene Glycol (**PG**) [Sigma, P-4347]
Triethyl citrate (**TEC**) [Aldrich, 109290]
Sodium Laurate (=dodecanoate) [Sigma, L9755]
Sodium Caprate (=decanoate) [Sigma, C4151]
Albumin-fluorescein isothiocyanate conjugate (**FITC-Albumin**), [Sigma, A9771]
Fluorescein isothiocyanate-dextran M.W. -40,000 (**FITC-Dextran** (**40K**)) [Sigma, FD40S]

### Contents and Preparation of Mixture "I":

2 g Sodium decanoate
2 g Sodium laurate
36 ml PG

All the above components (Sodium decanoate, Sodium laurate and PG) were mixed (vortexed) and heated (60°C) to dissolution. The resultant mixture "*1*" was then stored at 4°C.

### Preparation of FITC-Dextran Composition for gastric administration:

In 15ml test-tube, 5 mg FITC-Dextran (40K) at a final concentration of 0.5 mg/ml were dissolved in 2.5 ml of mixture "*1*" by heating to 60°C for ~2 hrs (intermittent mixing, as usual). 2.5 ml TEC was added and mixed in a rotary-shaker at 37°C, 60 rpm, for 5 hrs. The composition was stored at RT.

### Preparation of FITC- Albumin Composition for gastric administration:

In 15ml test-tube, 5 mg FITC- Albumin at a final concentration of 0.25 mg/ml were dissolved in 2.5 ml of mixture "*1*" by heating to 60°C for ∼2 hrs (intermittent mixing, as usual). 2.5 ml TEC was added and mixed in a rotary-shaker at 37°C, 60 rpm, for 5 hrs. The composition was stored at RT.

### Experimental Procedure:

Composition comprising FITC-Dextran (40K) at a final concentration of 0.5 mg/ml and of FITC-Albumin at a final concentration of 0.25 mg/ml were administered by gavage to mice which were than sacrificed after 5'-15':

FITC-Dextran composition was administered by gavage to a C57BL/6 male mouse (8-9 wks old (∼25g body weight)). After 5' the animal was sacrificed the stomach was removed and processed for frozen section according to the conventional procedure (see: JAMA 2005;294(24):3135-3137). 30µ-thick slides were visualized under light microscopy.

FITC-Albumin composition was administered by gavage to a C57BL/6 male mouse (8-9 wks old (∼25g body weight)). Non-formulated FITC-Albumin (PBS-dissolved) was administered to C57BL/6 male mouse (8-9 wks old (∼25g body weight)). Each mouse was sacrificed after 5' and 15' respectively, the abdominal wall of the mice was opened and the external side of the GI, where draining blood vessels and lymphatic vessels are located, was carefully viewed by a fluorescent stereoscope.

### Results:

Fig. 5 shows the crossing of FITC-Dextran (40K) from the gastric lumen into the lamina propria where blood vessels are located. Fig. 5 shows the translocation of the fluorescent composition from the gastric-pits' lumen into the lamina propria. Fig. 5 shows the cross-section through the gastric pits depicting FITC-Dextran pits' lumen (marked with * in Fig. 5) and in the lamina propria (marked with L in Fig. 5) where blood vessels are located. No staining is seen inside the cells, however, fine fluorescence can be seen between the cells, as expected from paracellular-crossing through tight-junctions.

Figs.6A-6B show the massive crossing of FITC-Albumin (-64 KDa) from the gastric lumen into the blood vessels that drain the stomach content and move it to the liver. Instead of continuous dark-red blood-vessels seen normally for non-formulated FITC-Albumin (Fig. 6A), when the composition of the invention comprising FITC-Albumin was administered into the stomach, the vessels look "pale" or even "discontinuous" (shown with arrows in Fig. 6B) due to major influx of the fluorescent protein from the gastric lumen into gastric-draining blood vessels.

Figs. 7A-7D illustrate the crossing of FITC-Albumin (-64 KDa) from the small-intestine (SI) lumen into the lymphatic vessels that drain the GI content and move it to the thoracic duct and then to the blood-stream. It is estimated that the composition comprising FITC-Albumin proceeds to the small-intestine (SI), and is shown to cross the SI epithelium via the basolateral space, as evident by the fluorescent-labeled lymphatic vessels and lymphatic nodules (shown with arrows in Figs. 7B and 7D).

## Claims

1. A composition comprising:
- at least one therapeutically active agent;
- at least one surface active agent being at least one of decanoic acid and lauric acid or any salt thereof;
- at least one ester of poly-carboxylic acid being triethyl-citrate; and
- a non-aqueous medium comprising propylene-glycol;
wherein said at least one therapeutically active agent is a macromolecule having a MW of between 0.5kDa to 200kDa selected from:
a protein, peptide, lipid, nucleic acid, glycosaminoglycan and any combination thereof, or
a hormone, growth factor, incretin, neurotrophic factor, anticoagulant, toxin, antibiotic, anti-fungal agent, antipathogenic agent, antigen, antibody, monoclonal antibody, antibody fragment, soluble receptor, immunomodulator, vitamin, antineoplastic agent, a cardiovascular agent, an antihypertensive agent, an agent used in the treatment of neurological disease, an agent capable of treating liver or kidney diseases, enzyme, gonadotropin, cytokine or any combination thereof.

2. A composition according to claim 1, for use in the gastric absorption of said at least one therapeutically active agent.

3. A composition according to any one of the preceding claims wherein said at least one therapeutically active agent is selected from the group consisting of insulin, C-peptide, erythropoietin (EPO), glucagon-like peptide 1 (GLP-I), melanocyte stimulating hormone (ALPHA-MSH), parathyroid hormone (PTH), parathyroid hormone amino acids 1-34 (PTH(1-34)), growth hormone, peptide YY amino acids 3-36 (PYY(3-36)), calcitonin, interleukin-2 (IL-2), alpha1-antitrypsin, granulocyte/monocyte colony stimulating factor (GM-CSF), granulocyte colony stimulating factor (G-CSF), T20, monoclonal antibodies such as anti-TNF antibodies, interferon alpha, interferon beta, interferon gamma, luteinizing hormone (LH), follicle- stimulating hormone (FSH), enkephalin, dalargin, kyotorphin, basic fibroblast growth factor (bFGF), hirudin, hirulog, luteinizing hormone releasing hormone (LHRH), brain-derived natriuretic peptide (BNP), glatiramer acetate, coagulation factor VIII; coagulation factor IX, neurotrophic factors, human chorionic gonadotropin (hCG), tissue plasminogen activator (TPA), oxytocin, and combinations thereof.

4. A composition according to any one of the preceding claims, wherein said at least one therapeutically active agent comprises at least 0.001 to 20% wt of the total weight of said composition.

5. A composition according to any one of the preceding claims, further comprising at least one ester of an aromatic carboxylic acid.

6. A composition according to any one of the preceding claims, wherein said at least one ester of poly-carboxylic acid comprises at least 5 to 90 %wt of total weight of said composition.

7. A composition according to any one of the preceding claims for use in the oral administration of said at least one therapeutically active agent.

8. A composition according to any of the preceding claims, further comprising ethyl-benzoate.

## Patentansprüche

1. Zusammensetzung, umfassend:
- mindestens einen therapeutischen Wirkstoff;
- mindestens ein oberflächenaktives Mittel, das mindestens eines von Decansäure und Laurinsäure oder ein beliebiges Salz davon ist;
- mindestens einen Ester einer Polycarbonsäure, der Triethylcitrat ist; und
- ein nicht-wässriges Medium, das Propylenglycol umfasst;
wobei der mindestens ein therapeutischer Wirkstoff ein Makromolekül ist, das ein MG zwischen 0,5 kDa und 200 kDa aufweist, ausgewählt aus:
einem Protein, einem Peptid, einem Lipid, einer Nukleinsäure, einem Glycosaminoglycan und einer beliebigen Kombination davon, oder
einem Hormon, einem Wachstumsfaktor, einem Inkretin, einem neurotrophen Faktor, einem Antikoagulans, einem Toxin, einem Antibiotikum, einem Antimykotikum, einem antipathogenen Mittel, einem Antigen, einem Antikörper, einem monoklonalen Antikörper, einem Antikörperfragment, einem löslichen Rezeptor, einem Immunmodulator, einem Vitamin, einem antineoplastischen Mittel, einem kardiovaskulären Mittel, einem blutdrucksenkenden Mittel, einem bei der Behandlung von neurologischen Erkrankungen verwendeten Mittel, einem Mittel, das zur Behandlung von Erkrankungen der Leber oder Nieren eingesetzt werden kann, einem Enzym, einem Gonadotropin, einem Zytokin oder einer beliebigen Kombination davon.

2. Zusammensetzung nach Anspruch 1 zur Verwendung bei der Aufnahme über den Magen mindestens eines therapeutischen Wirkstoffs.

3. Zusammensetzung nach einem der vorhergehenden Ansprüche, wobei mindestens ein therapeutischer Wirkstoff aus der Gruppe bestehend aus Insulin, C-Peptid, Erythropoietin (EPO), Glucagon-ähnliches Peptid 1 (GLP-1), Melanozytenstimulierendem Hormon (ALPHA-MSH), Parathormon (PTH), Parathormon-Aminosäuren 1-34 (PTH(1-34)), Wachstumshormon, Peptid YY-Aminosäuren 3-36 (PYY(3-36)), Calcitonin, Interleukin-2 (IL-2), alpha-1-Antitrypsin, Granulozyten-/Monozyten-Kolonie-stimulierendem Faktor (GM-CSF), Granulozyten-Kolonie-stimulierendem Faktor (G-CSF), T20, monoklonalen Antikörpern, wie Anti-TNF-Antikörpern, Interferon-alpha, Interferon-beta, Interferon-gamma, Luteinisierungshormon (LH), follikelstimulierendem Hormon (FSH), Enkephalin, Dalargin, Kyotorphin, basischem Fibroblastenwachstumsfaktor (bFGF), Hirudin, Hirulog, Luteinisierungshormon freisetzendem Hormon (LHRH), Gehirn-natriuretischem Peptid (BNP), Glatirameracetat, Gerinnungsfaktor VIII, Gerinnungsfaktor IX, neurotrophen Faktoren, humanem Choriongonadotropin (hCG), Gewebeplasminogenaktivator (TPA), Oxytocin und Kombinationen davon ausgewählt ist.

4. Zusammensetzung nach einem der vorhergehenden Ansprüche, wobei der mindestens eine therapeutische Wirkstoff mindestens 0,001 bis 20 Gew.% des Gesamtgewichts der Zusammensetzung umfasst.

5. Zusammensetzung nach einem der vorhergehenden Ansprüche, ferner umfassend mindestens einen Ester einer aromatischen Carbonsäure.

6. Zusammensetzung nach einem der vorhergehenden Ansprüche, wobei der mindestens eine Ester einer Polycarbonsäure mindestens 5 bis 90 Gew.% des Gesamtgewichts der Zusammensetzung umfasst.

7. Zusammensetzung nach einem der vorhergehenden Ansprüche zur Verwendung bei der oralen Verabreichung des mindestens einen therapeutischen Wirkstoffs.

8. Zusammensetzung nach einem der vorhergehenden Ansprüche, ferner umfassend Ethylbenzoat.

## Revendications

1. Composition comprenant :
- au moins un agent thérapeutiquement actif;
- au moins un agent tensio-actif étant au moins l'un parmi l'acide décanoïque et l'acide laurique ou l'un quelconque de leurs sels;
- au moins un ester d'acide polycarboxylique étant du citrate de triéthyle; et
- un milieu non aqueux comprenant du propylène glycol;
dans lequel ledit au moins un agent thérapeutiquement actif est une macromolécule ayant un poids moléculaire compris entre 0,5 kDa et 200 kDa choisie parmi :
une protéine, un peptide, un lipide, un acide nucléique, un glycosaminoglycane et toute combinaison de ceux-ci, ou
une hormone, un facteur de croissance, une incrétine, un facteur neurotrophique, un anticoagulant, une toxine, un antibiotique, un agent antifongique, un agent antipathogène, un antigène, un anticorps, un anticorps monoclonal, un fragment d'anticorps, un récepteur soluble, un immunomodulateur, une vitamine, un agent antinéoplasique, un agent cardiovasculaire, un agent antihypertenseur, un agent utilisé dans le traitement d'une maladie neurologique, un agent capable de traiter des maladies hépatiques ou rénales, une enzyme, une gonadotrophine, une cytokine ou toute combinaison de ceux-ci.

2. Composition selon la revendication 1, destinée à être utilisée dans l'absorption gastrique dudit au moins un agent thérapeutiquement actif.

3. Composition selon l'une quelconque des revendications précédentes, dans laquelle ledit au moins un agent thérapeutiquement actif est choisi dans le groupe constitué par l'insuline, le peptide C, l'érythropoïétine (EPO), le glucagon-like peptide-1 (GLP-1), l'hormone mélanotrope (ALPHA-MSH), l'hormone parathyroïdienne (PTH), les acides aminés 1-34 de l'hormone parathyroïdienne (PTH (1-34)), l'hormone de croissance, les acides aminés 3-36 du peptide YY (PYY (3-36)), la calcitonine, l'interleukine-2 (IL-2), l'alpha-1 antitrypsine, le facteur stimulant la formation de colonies de granulocytes et de monocytes (GM-CSF), le facteur stimulant les colonies de granulocytes (G-CSF), le T20, les anticorps monoclonaux tels que les anticorps anti-TNF, l'interféron alpha, l'interféron bêta, l'interféron gamma, l'hormone lutéinisante (LH), l'hormone folliculostimulante (FSH), l'enképhaline, la dalargine, la kyotorphine, le facteur de croissance de base des fibroblastes (bFGF), l'hirudine, l'hirulogue, l'hormone libérant l'hormone lutéinisante (LHRH), le peptide cérébral natriurétique (BNP), l'acétate de glatiramère, le facteur VIII de coagulation; le facteur IX de coagulation, les facteurs neurotrophiques, la gonadotrophine chorionique humaine (hCG), l'activateur de plasminogène tissulaire (TPA), l'ocytocine et leur combinaison.

4. Composition selon l'une quelconque des revendications précédentes, dans laquelle ledit au moins un agent thérapeutiquement actif comprend au moins 0,001 à 20% en poids du poids de ladite composition.

5. Composition selon l'une quelconque des revendications précédentes, comprenant en outre au moins un ester d'un acide carboxylique aromatique.

6. Composition selon l'une quelconque des revendications précédentes, dans laquelle ledit au moins un ester d'acide polycarboxylique comprend au moins 5 à 90% en poids du poids total de ladite composition.

7. Composition selon l'une quelconque des revendications précédentes à utiliser dans l'administration orale dudit au moins un agent thérapeutiquement actif.

8. Composition selon l'une quelconque des revendications précédentes, comprenant en outre du benzoate d'éthyle.
